# EUROPEAN PATENT SPECIFICATION

(11) **EP 4 132 619 B1**
(45) Date of publication and mention of the grant of the patent: **18.02.2026**
(21) Application number: 21784630.2
(22) Date of filing: 09.04.2021
(51) Int. Cl.: A61M 16/08

(54) **A RESPIRATORY CONDUIT**
BEATMUNGSLEITUNG
CONDUIT RESPIRATOIRE

(30) Priority: 09.04.2020 US 202063007633 P
(43) Date of publication of application: 15.02.2023
(73) Proprietor: Fisher & Paykel Healthcare Limited, East Tamaki Auckland 2013 (NZ)
(72) Inventor: NEWELL, Caoimhe Marie, 2013 Auckland (NZ); RUTLEDGE, Timothy Richard, 2013 Auckland (NZ); WHITE, Craig Karl, 2013 Auckland (NZ)
(74) Representative: Ström & Gulliksson AB
(86) International application number: PCT/NZ2021/050061
(87) International publication number: WO 2021/206568

(56) References cited:
- EP-A2- 0 621 050
- EP-A2- 0 672 430
- WO-A1-03/022342
- WO-A2-2013/002655
- US-A- 6 078 730
- US-A1- 2017 252 529
- US-A1- 2017 252 529
- US-A1- 2019 388 640
- US-B1- 6 536 428

## Description

### FIELD OF THE INVENTION

This invention relates to a respiratory conduit.

### BACKGROUND

Medical devices or equipment (for example, in a hospital) which are to be reused may require cleaning and disinfection after each procedure or patient use. The level of cleaning and disinfection required depends on the use of the medical device and the extent that it is in contact with a patient or fluids from the patient. For example, environmental equipment that does not contact a patient such as flow generators, ventilators, anaesthesia machines and humidifiers may have an accepted lower standard for cleaning and disinfection than the level required for equipment that comes into skin contact with a patient. Skin-contacting equipment can require cleaning between patients, for example, a relatively low-level disinfection involving cleaning with a disinfecting agent to destroy pathogens.

Skin contacting equipment may include the external, skin contacting surfaces of respiratory conduits such as inspiratory and expiratory tubes. For such conduits to be reused for multiple patients the outer surface is preferably cleaned to a suitable standard after each use. The outer surface of respiratory conduits such as inspiratory tubes is often corrugated or undulating to, for example, enhance the flexibility of the tube. Sufficient cleaning and disinfection of these surfaces can be difficult as the recesses between corrugation ridges can trap dirt and pathogens and be difficult to access to adequately clean. To avoid difficult cleaning, new respiratory conduits are often used for each patient then discarded after a single use. Single use of products undesirably increases cost and the level of waste.

EP0672430A2 shows examples of a humidifier conduit, WO2013/002655A2 shows examples of medical tubing and US2017/252529A1 shows examples of an air delivery conduit.

It is an object of at least preferred embodiments of the present invention to address one or more of the above-mentioned disadvantages and/or to at least provide the public with a useful alternative.

In this specification where reference has been made to patent specifications, other external documents, or other sources of information, this is generally to provide a context for discussing features of the invention. Unless specifically stated otherwise, reference to such external documents or sources of information is not to be construed as an admission that such documents or such sources of information, in any jurisdiction, are prior art or form part of the common general knowledge in the art.

### SUMMARY OF THE INVENTION

The invention relates to a respiratory conduit comprising a hollow tube having an inlet, an outlet, and a longitudinal axis, and defining a fluid flow path. Along at least a length of the tube, an outer surface of the tube comprises alternate ridges and grooves. The respiratory conduit also comprises a sheath surrounding at least a portion of the length of the tube, the sheath comprising alternate attachment portions contacting the ridges of the tube and bridging portions that extend across the grooves of the tube. Each bridging portion has a span length being the distance in the longitudinal direction between adjacent attachment portions, and a depth being the maximum distance in a radial direction between a point on the bridging portion and a point on one of the adjoining attachment portions, wherein the depth of the bridging portion is greater than zero. The ratio of the bridging portion span length to its depth is less than or equal to a predetermined ratio upper limit. The predetermined ratio upper limit is about 2:1. The ratio of the bridging portion span length to its depth is greater than or equal to a predetermined ratio lower limit. The predetermined ratio lower limit is about 2:0.1.

Preferably, the conduit is in a rest state, such that the conduit is not extended, flexed or compressed. In some forms, the conduit is extendible/stretchable from a rest state and wherein in an extended state the bridging portion depth is decreased from the rest state. Optionally, the conduit is biased toward the rest stage so as to return to the rest state after being in the extended state.

In some forms, the sheath has a yield strain of at least 45% in the longitudinal direction.

In some forms, the depth of the bridging portion is less than a depth of the respective groove across which the bridging portion extends. Preferably, the bridging portion extends across the respective groove in a spaced apart relationship such that the outer surface of the groove and an inner surface of the respective bridging portion define a cavity. In some forms, the cavity forms a sealed air pocket.

In some forms, the point on the bridging portion where the depth is maximum is located at a middle point between adjacent ridges. Preferably, an outer surface of the bridging portion is concave. In some forms, the depth of the bridging portion is substantially the same along substantially all of the span of the bridging portion. In some forms, an internal diameter of the bridging portion is substantially constant along the span of the bridging portion. Preferably, the bridging portions are substantially arcuate.

In some forms, a minimum diameter of each bridging portion is less than a minimum diameter of the attachment portions, such that the bridging portions extend inward from the attachment portions. In other forms, a maximum diameter of each bridging portion is greater than a maximum diameter of the attachment portions, such that the bridging portions protrude outward from the attachment portions.

Preferably, the sheath is tightly fitted to the tube such that the attachment portions contact an outer surface of at least a portion of the tube ridges and at least partially adopt a profile of the tube ridges. In some forms, the attachment portions are attached to the tube at the tube ridges. Optionally, the attachment portions are bonded to the tube at the tube ridges. For example, each attachment portion may be bonded to the tube along substantially the length of the attachment portion. In some forms, the attachment portions are bonded to the tube at or near edges of the attachment portions, adjacent respective bridging portions.

In some forms, the attachment portion has a predetermined length between two transition regions where the attachment portion transitions to respective adjoining bridging portions. Preferably, the attachment portions are arcuate and the predetermined attachment length is an arc length. Optionally, the predetermined attachment length is more than 0 mm and less than or equal to about 4 mm. In some forms, a tangent to the outer surface of the attachment portion at each transition region has an angle of between about 10 degrees and about 70 degrees relative to the longitudinal axis of the tube.

In some forms, each ridge or a portion thereof has a predetermined radius of curvature greater than 0 mm and less than or equal to about 3 mm.

Optionally, the sheath has a substantially uniform wall thickness in a rest state of between about 100 µm and about 250 µm. In some forms, the sheath and/or the tube comprises a thermoplastic. Preferably, the sheath and/or the tube comprises a low- density polyethylene (LDPE). Optionally, wherein the sheath is transparent.

In some forms, the predetermined ratio of the bridging portion span length to its depth is selected to minimise outwards buckling of the sheath. Optionally, the predetermined ratio of the bridging portion span length to its depth is selected to enable low level disinfection of an exterior surface of the sheath.

In some forms, the tube is corrugated. Preferably, the ridges and grooves comprise a series of annular ridges and grooves. In some forms, the ridges and grooves are helical. Alternatively, the tube is formed from one or more spirally wound components. In some forms, the tube comprises two or more spirally wound components. Optionally, the two or more spirally wound components comprise one or more of: an elongate hollow body, and an elongate structural component. In some forms, the elongate structural component comprises heating elements, sensing elements, or both heating and sensing elements.

In some forms, the respiratory conduit also comprises an internal heating element.

In a second aspect not independently claimed the disclosure provides a respiratory conduit comprising a hollow tube having an inlet, an outlet, and a longitudinal axis, and defining a gas flow path. Along at least a length of the tube, an outer surface of the tube comprises alternate ridges and grooves. The respiratory conduit also comprises a sheath surrounding at least a portion of the length of the tube. The sheath comprises alternate attachment portions attached to the ridges of the tube and bridging portions that extend across the grooves of the tube. Each attachment portion is bonded to at least one transition region on the respective ridge of the tube, the transition region being where the attachment portion transitions to an adjacent bridging portion, wherein, (i) the ridge, or a portion thereof, has a predetermined radius of curvature and/or (ii) a surface of the sheath at the transition region defines a tangent, the tangent being at a predetermined angle relative to a longitudinal axis of the tube and/or (iii) the attachment portion has a predetermined length between respective transition regions on the respective ridge.

In some forms, the ridge or a portion thereof has a predetermined radius of curvature greater than 0 mm and less than or equal to about 3 mm.

In some forms, a surface of the sheath at the transition region defines a tangent, the tangent having a predetermined angle, relative to the longitudinal axis of the tube, of between 10 and 70 degrees.

In some forms, the attachment portion contacts the tube along a predetermined attachment length between respective transition regions and the predetermined attachment length is more than 0 mm and less than or equal to about 4 mm. Preferably, the predetermined attachment length is between about 2 mm and about 4 mm.

In some forms, the conduit is in a rest state, such that the conduit is not extended, flexed or compressed.

Optionally, the attachment portions are arcuate and the predetermined attachment length is an arc length. Preferably, an outer surface of the attachment portions is convex.

In some forms, the sheath contacts the ridge along substantially the whole of the attachment length. Optionally, the attachment portion is bonded to the respective ridge along substantially the whole of the attachment length. In some forms, the attachment portion is bonded to the respective ridge at discrete transition bonding locations, located symmetrically on either side of the ridge. Optionally, the attachment portion is bonded to a peak of the respective ridge.

Preferably, the sheath has a substantially uniform wall thickness of between about 100 µm and about 250 µm in a rest state.

In some forms, each bridging portion has a pre-determined span length that is the distance in the longitudinal direction between adjoining adherent portions. Preferably, each bridging portion has a pre-determined depth that is the maximum distance in a radial direction between a point on the bridging portion and a point on the attachment portion; wherein the depth of the bridging portion is greater than zero. Optionally, the pre-determined bridging portion depth dip is less than or equal to about 1 mm.

In some forms, each bridging portion has a predetermined ratio of the bridging portion span length to its depth. Optionally, the predetermined ratio is between about 2:0.1 and about 2:1. In some forms, the predetermined ratio of the bridging portion span length to its depth is selected to minimise outwards buckling of the sheath. Optionally, the predetermined ratio of the bridging portion span length to its depth is selected to enable low level disinfection of an exterior surface of the sheath.

In some forms, the sheath has a yield strain of at least 45% in the longitudinal direction.

In some forms, the depth of the bridging portion is less than a depth of the respective groove. Optionally, the bridging portion extends across the respective groove in a spaced apart relationship such that the outer surface of the groove and an inner surface of the respective bridging portion define a cavity. Preferably, the cavity forms a sealed air pocket.

In some forms, the tube is corrugated. Preferably, the ridges and grooves comprise a series of annular ridges and grooves. In some forms, the ridges and grooves are helical. Alternatively, the tube is formed from one or more spirally wound components. In some forms, the tube comprises two or more spirally wound components. Optionally, the two or more spirally wound components comprise one or more of: an elongate hollow body, and an elongate structural component. In some forms, the elongate structural component comprises heating elements, sensing elements, or both heating and sensing elements.

In some forms, the respiratory conduit further comprises an internal heating element.

In a third aspect not independently claimed the disclosure provides a respiratory conduit comprising a hollow, inner tube having an inlet, an outlet, and a fluid flow path defined between the inlet and the outlet. Along at least a length of the tube, an outer surface of the tube comprises alternate ridges and grooves. The respiratory conduit also comprises an outer sheath surrounding at least a portion of the length of the tube, the sheath comprising alternate attachment portions contacting and conforming to the external surface of the ridges of the tube. The ratio between the largest diameter of the inner tube, x, and the diameter of the outer sheath is between x:x and 0.9:x

Preferably, at least a portion of the sheath has a diameter less than the largest diameter of the conduit. Preferably, the conduit is in a rest state, such that the conduit is not extended, flexed or compressed.

In some forms, the sheath is tightly fitted to the tube such that the attachment portions contact an outer surface of at least a portion of the tube ridges and at least partially adopt a profile of the tube ridges. Optionally, the attachment portions are attached to the tube at the tube ridges. In some forms, the attachment portions are bonded to the tube at the tube ridges. Optionally, the attachment portions are bonded to the tube along substantially the length of the attachment portion. In some forms, the attachment portions are bonded to the tube at or near edges of the attachment portions, adjacent respective bridging portions. In some forms, the attachment portion has a predetermined length between two transition regions where the attachment portion transitions to respective adjoining bridging portions.

In some forms, the attachment portions are arcuate and the predetermined attachment length is an arc length. Optionally, the predetermined attachment length is more than 0 mm and less than or equal to about 4 mm. In some forms, a tangent to the outer surface of the attachment portion at each transition region has an angle of between about 10 degrees and about 70 degrees relative to the longitudinal axis of the tube.

Preferably, each ridge or a portion thereof has a predetermined radius of curvature greater than 0 mm and less than or equal to about 3 mm.

In some forms, the sheath has a substantially uniform wall thickness in a rest state of between about 100 µm and about 250 µm.

In some forms, the sheath and/or the tube comprises a thermoplastic. Preferably, the sheath and/or the tube comprises a low-density polyethylene (LDPE). Optionally, the sheath is transparent.

In some forms, the tube is corrugated. Preferably, the ridges and grooves comprise a series of annular ridges and grooves. Optionally, the ridges and grooves are helical. Alternatively, the tube is formed from one or more spirally wound components. In some forms, the tube comprises two or more spirally wound components. Optionally, the two or more spirally wound components comprise one or more of: an elongate hollow body, and an elongate structural component. In some forms, the elongate structural component comprises heating elements, sensing elements, or both heating and sensing elements.

In some forms, the respiratory conduit further comprises an internal heating element.

In a fourth aspect not independently claimed the disclosure provides a multi-use respiratory conduit for use in a medical procedure. The conduit comprises a respiratory conduit as described herein according to any one of the first to third aspects of the disclosure and in which the tube inlet is configured for connection to a humidifier and the tube outlet is configured for connection to a patient interface.

In some forms, a connector port at or near the tube outlet, for receiving a sensor. Preferably, the port is configured to enable exposure of the sensor to a gases flow through the conduit. In some forms, the sensor is a sensor for sensing one or more of temperature, humidity, and/or flow.

In some forms, the tube comprises a heating element. Preferably, the heating element is located in a side wall of the inner tube. Optionally, the heating element comprises one or more heating wires. Preferably, the heating element is provided in the gas flow path.

In some forms, the tube inlet comprises a connector adapted to removably engage with an upstream respiratory system component.

In some forms, the tube outlet comprises a connector adapted to removably engage with a downstream respiratory system component.

In some forms, the downstream respiratory system component comprises a patient interface. Preferably, the patient interface comprises a filter, and wherein the outlet end of the conduit removably engages with an inlet port of the filter. Optionally, the patient interface is non-sealing. For example, the patient interface may comprise a nasal cannula.

In some forms, the patient interface is for single-patient use.

In some forms, the respiratory conduit may also comprise at least one of a temperature sensor and a gas flow rate sensor located in the tube outlet.

In a fifth aspect not independently claimed the disclosure provides a kit for a respiratory system, the kit comprising a respiratory conduit according to any one of the first, second or third aspects of the disclosure; at least one of: a dry line, a humidifier, a patient interface, and/or a filter.

In some forms, the respiratory conduit comprises a heating element. Optionally, the heating element is provided in the gas flow path of the conduit. In some forms, the heating element comprises heating wires.

In some forms, the kit comprises a first package comprising the dry line, humidifier and the respiratory conduit. Preferably, the components in the first package are for multi- patient use. Optionally, the components in the first package are for use within a single day.

In some forms, the kit comprises a second package comprising a patient interface. Optionally, the patient interface comprises a filter.

In some forms, the kit comprises a clip to releasably secure the respiratory conduit to clothing or bedding.

Optionally, components in the second package are for single-patient use.

In some forms, the patient interface is non-sealing. For example, the patient interface may comprise a nasal cannula.

In a sixth aspect of the disclosure, not independently claimed, the respiratory system comprises a flow source; a respiratory conduit according to any one of the first, second or third aspect of the disclosure; and a patient interface.

In some forms, the respiratory conduit is for multi-patient use. Preferably, the respiratory conduit is for use within a single day.

Optionally, the patient interface is for single patient use.

In some forms, the patient interface is non-sealing. For example, the patient interface may comprise a nasal cannula.

In some forms, the system comprises a high flow system. Preferably, the flow source provides flow between 20-90 L/min.

In some forms, the system comprises a humidifier configured to humidify a flow of gases from the flow source to the patient interface via the respiratory conduit.

In some forms, the respiratory system is to be used in a medical procedure. Optionally, the respiratory system is to be used in a sedation and/or anaesthetic procedure. In some forms, the respiratory system is to be used in an environment wherein a portion of the environment is sterile.

In a seventh aspect not independently claimed the disclosure provides a method for forming a respiratory conduit according to any one of the first, second or third aspects of the disclosure. The method comprises the steps of: forming a tube with an outer surface having, along at least a length of the tube, alternate ridges and grooves; extruding a sheath around the length of the tube comprising the alternate ridges and grooves; and installing a connector to an end of the tube.

Optionally, the tube is corrugated and the step of forming the tube comprises forming corrugations via vacuum corrugation or pressure corrugation. The corrugations may comprise alternate ridges and grooves. The alternate ridges may be annular along at least a portion of the length of the tube, or they may be helical along at least a portion of the tube. The alternate grooves may be annular along at least a portion of the length of the tube, or they may be helical along at least a portion of the tube.

In some forms, the step of extruding the sheath comprises passing the tube through a cross-head die.

Optionally, the step of extruding the sheath comprises bonding the sheath to the ridges of the tube.

In some forms, the step of extruding the sheath comprising fitting the sheath tightly about the tube to cause portions of the sheath to at least partially adopt a profile of the ridges.

In some forms, the method also includes the step of engaging the connector with the respective end of the tube such that the end of the tube receives a portion of the connector without affecting the integrity of the sheath.

Optionally, the sheath comprises a hydrophobic material and/or an anti-microbial material and/or a material with a predetermined tackiness. Preferably, the predetermined tackiness has a coefficient of friction of less than 0.6.

In some forms, the sheath has a substantially uniform thickness after extrusion of between about 100 µm and about 250 µm.

In an eighth aspect not independently claimed the disclosure relates to a respiratory conduit comprising a hollow tube having an inlet, an outlet, and a longitudinal axis, and defining a fluid flow path. Along at least a length of the tube, an outer surface of the tube comprises alternate ridges and grooves, the ridges having an outer diameter. The respiratory conduit also comprises a sheath surrounding at least a portion of the length of the tube, the sheath comprising alternate attachment portions contacting the ridges of the tube and bridging portions that extend across the grooves of the tube. Each bridging portion has a depth being the maximum distance in a radial direction between a point on the bridging portion and a point on one of the adjoining attachment portions, wherein the depth of the bridging portion is between about **2% and about 15%** of the outer diameter of the ridges.

In some forms, the depth of the bridging portion is less than about 10% of the outer diameter of the ridges.

The respiratory conduit of the eighth aspect may have any one or more of the features described above in relation to the other aspects.

This disclsoure may also be said broadly to consist in the parts, elements and features referred to or indicated in the specification of the application, individually or collectively, and any or all combinations of any two or more said parts, elements or features. Where specific integers are mentioned herein which have known equivalents in the art to which this disclosure relates, such known equivalents are deemed to be incorporated herein as if individually described.

The term 'comprising' as used in this specification and claims means 'consisting at least in part of'. When interpreting statements in this specification and claims that include the term 'comprising', other features besides those prefaced by this term can also be present. Related terms such as 'comprise' and 'comprised' are to be interpreted in a similar manner.

It is intended that reference to a range of numbers disclosed herein (for example, 1 to 10) also incorporates reference to all rational numbers within that range and any range of rational numbers within that range (for example, 1 to 6, 1.5 to 5.5 and 3.1 to 10).

Therefore, all sub-ranges of all ranges expressly disclosed herein are hereby expressly disclosed.

As used herein the term '(s)' following a noun means the plural and/or singular form of that noun. As used herein the term 'and/or' means 'and' or 'or', or where the context allows, both.

### BRIEF DESCRIPTION OF THE DRAWINGS

The present invention will now be described by way of example only and with reference to the accompanying drawings in which:
Figure 1 is a schematic illustrating an exemplary respiratory system;
Figure 2 is a partial side view of a portion of a conduit according to one embodiment of the present invention, illustrating the attachment of the sheath to the inner tube;
Figure 3 is a partial side perspective view of a length of one embodiment inner tube having ridges and grooves;
Figure 4 is a side section view of the tube of Figure 3, taken through a plane along the longitudinal axis;
Figure 5 is a partial side detail view of the inner tube of Figures 3 and 4 showing the profile of the ridges and grooves;
Figure 6 is a partial side detail view showing the profile of the ridges and grooves for an alternative form of inner tube;
Figure 7 is a cut-away partial perspective view of a portion of an alternative inner tube having a helical bead;
Figure 8 is a cut-away partial perspective view of a portion of an alternative inner tube having two spirally wound members;
Figure 9 is a cut-away partial perspective view of a portion of an alternative inner tube having three spirally wound members;
Figure 10 is a schematic partial section view showing the cross-sectional wall profile for one form of inner tube having a spirally wound hollow member and a spirally wound structural member.
Figure 11 is a detail perspective section view of the hollow spirally wound member and spirally wound structural member of Figure 10;
Figure 12 is a side view of a conduit according to one embodiment of the present disclosure;
Figure 13 is an enlarged view of the detail A in Figure 12;
Figure 14 is a partial side view of an alternative conduit utilising the inner tube of Figure 6;
Figures 15(i) to 15(iii) show various possible tangent angles at the transition point for the transition from the sheath attachment portion to the bridging portion, where Figure 15(i) shows a steep angle, Figure (ii) shows a more desirable angle, and Figure 15(iii) shows a shallow tangent angle;
Figures 16(i) and 16(ii) illustrate alternative shapes for sheath bridging portions for the inner tube of Figure 2, where the bridging portion of Figure 16(i) has a shorter constant depth region than the bridging portion of Figure 16(ii);
Figure 17 is a partial section view illustrating the wall profile for another form of conduit having a sheath with a sinuous profile.

In these figures, like reference numbers are used for different embodiments to indicate like features, but with the addition of a multiple of 100.

### DETAILED DESCRIPTION OF A PREFERRED EMBODIMENT

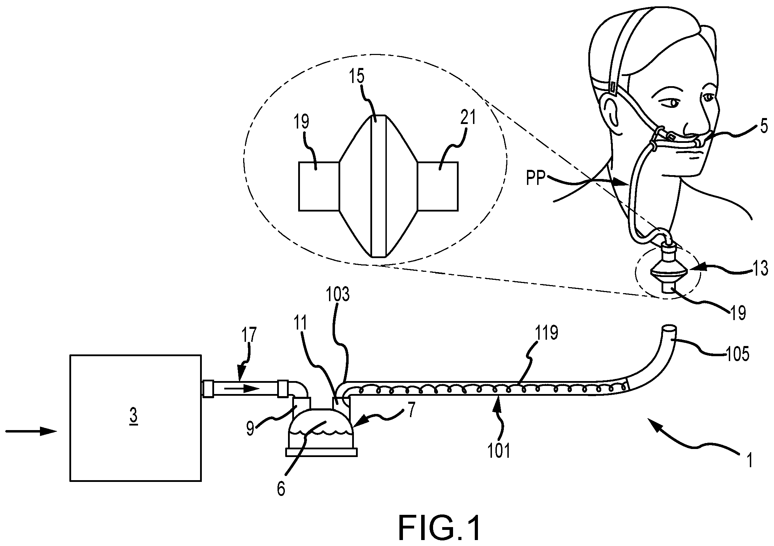

Figure 1 shows an exemplary respiratory therapy system 1 for delivering pressurised gases to a patient PP. The system 1 may be suitable for use in a medical procedure, such as during a sedation and/or anaesthetic procedure and is especially suitable for use in an environment, such as a surgical environment or an emergency room, where at least a portion of the environment may be sterile and equipment used may be sterile, easily cleaned, or at least configured to reduce the risk of bacterial build up. The system 1 may also be suitable for use in areas of a medical facility or environment where it is desirable to have medical devices and/or components configured for multiple patient use, for example in ambulances and emergency department, where patient turnover is generally high.

The system 1 comprises a flow source 3, such as a wall supply of gasses fluidly connected to a respiratory conduit 101, and a patient interface 5. The flow source 3 typically provides a gases flow comprising a mix of air and oxygen.

The system 1 shown in Figure 1 is a humidifying circuit comprising a humidifier 7 downstream of the flow source 3. The humidifier 7 comprises a humidification chamber 6, an inlet 9 for receiving dry air from the flow source via a dry line 17, and an outlet 11 for delivering humidified air to the patient PP via the conduit 101 and patient interface 5. The humidifier 7 may be of a type known in the art, for example, comprising a humidification chamber 6 with a volume of water between the humidifier inlet 9 and outlet 11 and a heating element to heat the water, for humidifying gases flowing through the humidification chamber 6.

In the embodiment shown, the patient interface 5 is a non-sealing nasal cannula, and the flow source is typically arranged to provide a gas flow to the patient PP of between 20-90 L/min. Different flow rates may be used for different patients. For example, lower flow rates may be more suitable for infants. The patient interface and flow source may also be used to provide high flow gas to the patient. However, it will be appreciated that alternative non-sealing or sealing patient interfaces such as oral or full-face masks may be used, and such alternative patient interfaces may require different gas flow rates.

The inspiratory conduit 101 is described in more detail below. Unless specified, the embodiments as described herein are described in relation to a conduit in a normal rest state as opposed to an extended, compressed, or flexed state where the conduit is subject to an element of deformation.

The conduit 101 comprises an inlet 103 and an outlet 105. The inlet 103 removably and fluidly connects to the outlet 11 of the humidifier 7, and the outlet 105 removably and fluidly connects to the patient interface 5. In some forms, the conduit may be a single use conduit, such as for single-patient use or for use on a single day. In other forms, the conduit may be for multi-patient use or for use across multiple days. For multi-patient or multi-day use, the conduit is typically configured so that the outlet 105 removably and fluidly connects to an optional filter 13 upstream of the patient interface. For example, the conduit inlet 103 and/or outlet 105 may comprise a connector for removably engaging with another component in the respiratory system such as a filter.

The optional filter 13 may be a high-efficiency particulate arrestance (HEPA) filter or any other suitable filter. The filter 13 shown comprises an inlet port 19 an outlet port 21, and a generally cylindrical filter housing 15 with an enlarged central body portion that tapers toward the inlet and outlet ports 19, 21 respectively. The enlarged central portion of the filter housing 15 contains suitable filtration material through with gases from the conduit 101 pass to reach the patient interface 5. The filtration material may comprise pleated paper, nano-fibres, or any other suitable filtration material, including sock filters, stacked disc filters, spiral filters, block(s) of filter material, a disc or discs of filter material with streams of filter material to free flow from or off the disc in fluid flow, for example. The filter 13 captures and prevents downstream passage therethrough of particulates, bacteria and/or other infectious material from the inspiratory conduit 101 to the patient PP, and importantly captures and prevents upstream passage therethrough of bacteria and/or other infectious material from the patient to the inspiratory conduit 101. The filter 13 is preferably a single-use component that is replaced for each patient to enable reuse of the conduit 101 between multiple patients.

The conduit 101 may comprise a port at or near the outlet 105, for receiving a sensor to measure a property of gas flow through the conduit. For example, the sensor may be configured to measure one or more of temperature, humidity, and/or flow rate. In some forms, the sensor is a probe, such as a flow probe or temperature probe. The port is configured to expose the sensor to the gas flow through the conduit 101.

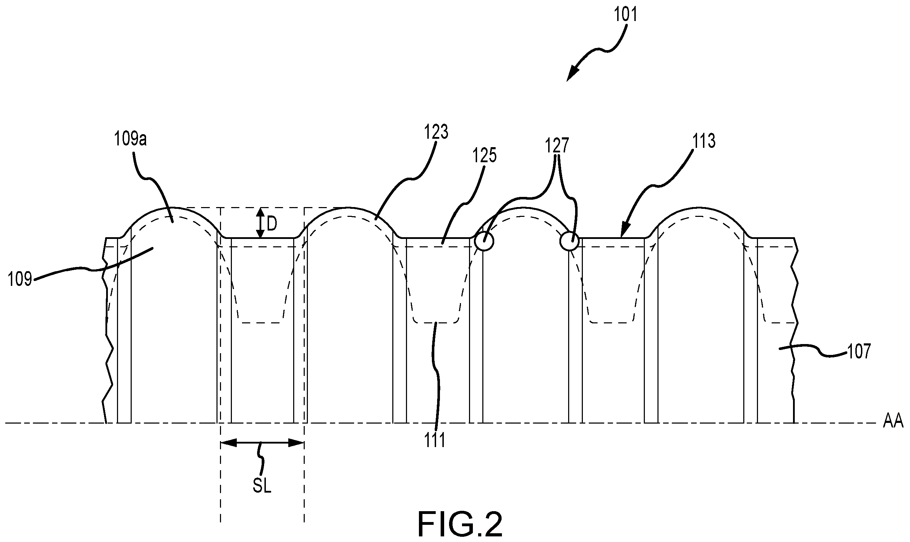

Figure 2 shows a portion of a respiratory conduit 101 in accordance with one form of the present invention. The conduit 101 comprises an inner hollow tube 107 for conveying gases along the conduit 101, and an outer sheath 113. The inner tube 107 has an inlet 103, an outlet 105, and a longitudinal axis AA, defining a fluid flow path from the inlet 103 to the outlet 105. The fluid flow path is generally parallel with the longitudinal axis AA. The inner tube 107 is a flexible tube, with at least a length of the inner tube 107 having an outer surface comprising alternate/successive ridges 109 and grooves 111.

Herein, the conduit 101 will be described in relation to the cross-sectional profile of the conduit taken along a longitudinal plane. Thus it should be understood that 'length' is used to refer to a dimension generally in the longitudinal direction of the conduit, and the terms 'height' or similar refer to a radial distance from the longitudinal axis AA.

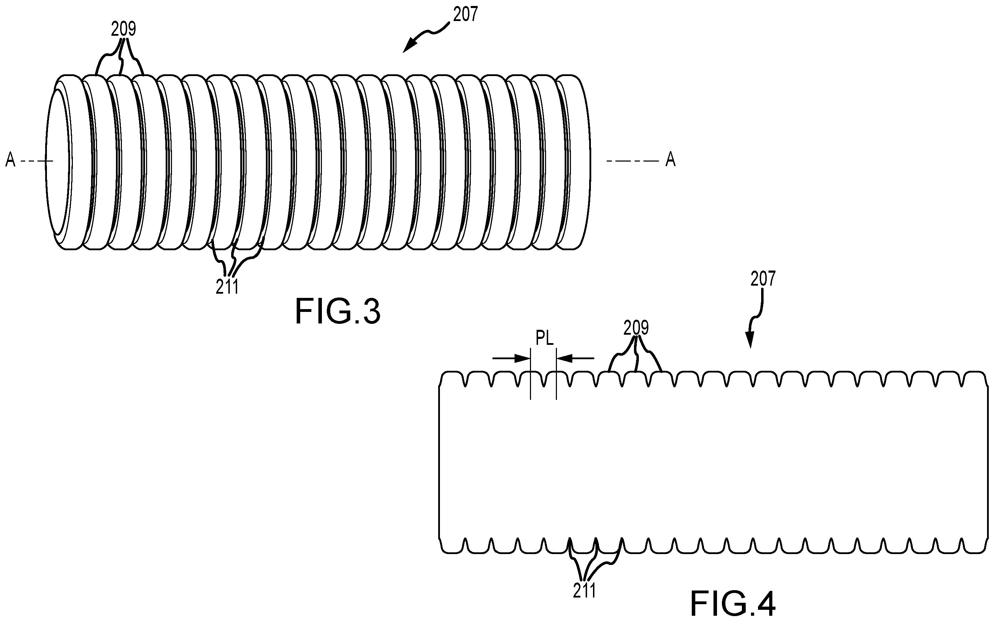

Figures 3 to 5 illustrate a length of one form of inner tube 107. The inner tube 207 is corrugated, having a series of annular ridges 209 that define annular grooves 211 therebetween. Each ridge 209 has an apex 209a at a maximum distance from the longitudinal axis AA, and adjoining upstream and downstream angled side walls 209b diverging from the apex. The side walls transition to a groove minima, which is the minimum distance between the longitudinal axis AA of the tube and the groove 211.

The conduit, including the inner hollow tube 107 for conveying gases along the conduit 101, and the outer sheath 113 may have a length defining a fluid flow path from the inlet 103 to the outlet 105. The length of at least the inner tube 107 defined between the inlet 103 and the outlet 105 may be between 0.5 and 3.5 m. The length is preferably greater than 2m. More preferably, the length of the inner tube 107 is about 2.4m.

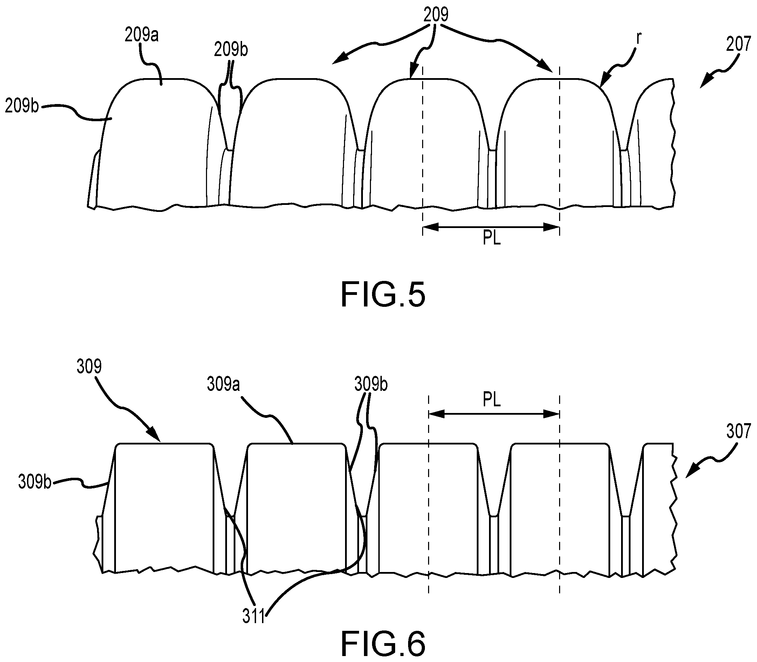

Referring to Figure 5, the transition between the apex of the ridge 209a and the ridge side walls 209b is preferably a smooth, rounded transition, for example. with a radius of curvature r that is greater than 0 mm and less than or equal to about 4 mm. In the embodiment of Figures 3 to 5, the ridges 209 have a cross sectional profile with a substantially flat or blunt section 209a at the apex of the ridge and a curved transition to the side walls 209b with a radius of between about 0.5 mm and about 3 mm. In one embodiment the radius is about 1 mm. The ridges have a pitch, PL of between about 1 mm and about 10 mm measured in the longitudinal direction as the distance between the centres of the apexes 209a of adjacent ridges 209 when the conduit 101 is in an unflexed, rest state. Preferably, the ridges have a pitch, PL between about 2 mm and about 6 mm. In one embodiment the ridges have a pitch, PL of about 4.5 mm. In an alternative embodiment, the ridges have a pitch, PL of about 3.5 mm.

The apex of the ridges 209 define a maximum outer diameter of the inner tube 207 of between about 10 mm and about 35 mm, preferably between about 10 mm and about 30 mm. In one embodiment, the apex of the ridges 209 define a maximum outer diameter of the inner tube 207 of about 25 mm. In an alternative embodiment the maximum outer diameter defined is about 18 mm. The minima of each groove 211 defines a minimum inner diameter of the corrugated section of tube of between about 5 mm and about 30 mm, preferably between about 10 mm and about 25 mm. In one embodiment, the minima of each groove 211 defines an inner diameter of the inner tube 207 of about 12 mm. In an alternative embodiment, the minima of each groove 211 defines an inner diameter of the inner tube 207 of about 22 mm

The inner tube 207 has an overall wall thickness measured from the inner diameter of the grooves 211 to the outer diameter of the apex of the ridges 209 of between about 0.1 mm and about 10 mm. In one embodiment the wall thickness is between about 0.2 mm and about 1.5 mm, preferably about 0.3 mm to about 0.4 mm. In an alternative embodiment, the wall thickness is between about 2 mm and about 6 mm.

In one embodiment, the minima of each groove 211 defines a minimum outer diameter of the corrugated section of tube of about 20 mm, however, other dimensions are envisaged. The apex of the ridges may be substantially flat/blunt, outwardly curved, or outwardly angled to form an angular apex. Alternatively, the ridges of the inner tube may have a sharper, more angular transition to the groove, such that the side walls 309b meet the ridge apex 309a and groove minima in an angular arrangement. Figure 6 illustrates an example of one embodiment of inner tube 207 with a sharper angle between the ridge apex 309a and the ridge side walls 309b. This embodiment is generally less desirable than the embodiment of Figures 3 to 5.

The inner tube 107 may comprise one or more heating wires 119 (Figure 1) to prevent condensation forming in the tube as the gases move along the tube, by minimising any drop in temperature. The heating wires 119 may be attached to or integral with the wall of the inner tube, or they may be provided in the hollow of the tube, in the gases flow path.

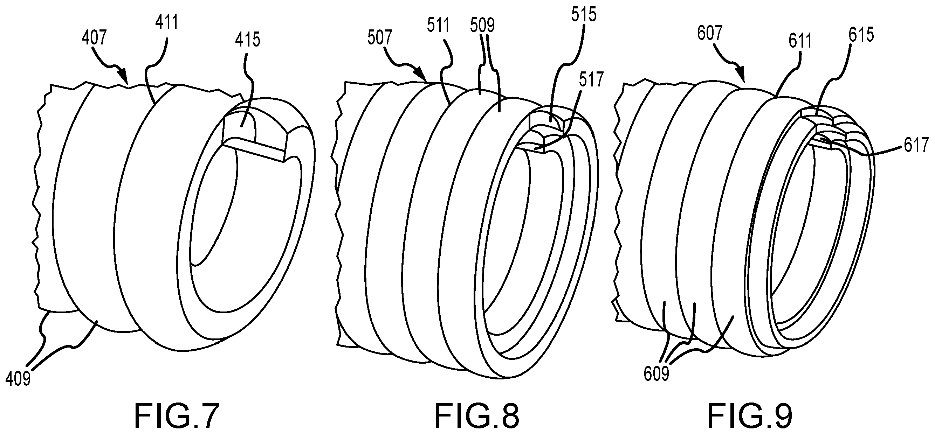

Rather than having corrugations formed by annular ridges and grooves, the ridges and grooves of the inner tube may be formed by one or more helical beads. Figures 7 to 9 illustrate exemplary inner tubes 407, 507, 607 with the ridges 409, 509, 609 and grooves 411, 511, 611 of the tube outer surface formed by a single hollow body helical bead 415, 515, 615. Although the single spirally wound bead 415, 515, 615 is continuous along the length of the tube, it forms a plurality of ridges and grooves in the longitudinal direction of the tube. In other embodiments, the ridges and grooves may be formed from two of more spirally wound components. The tubes illustrated in Figure 7 to 9 comprise outwardly curved ridges 409, 509, 609.

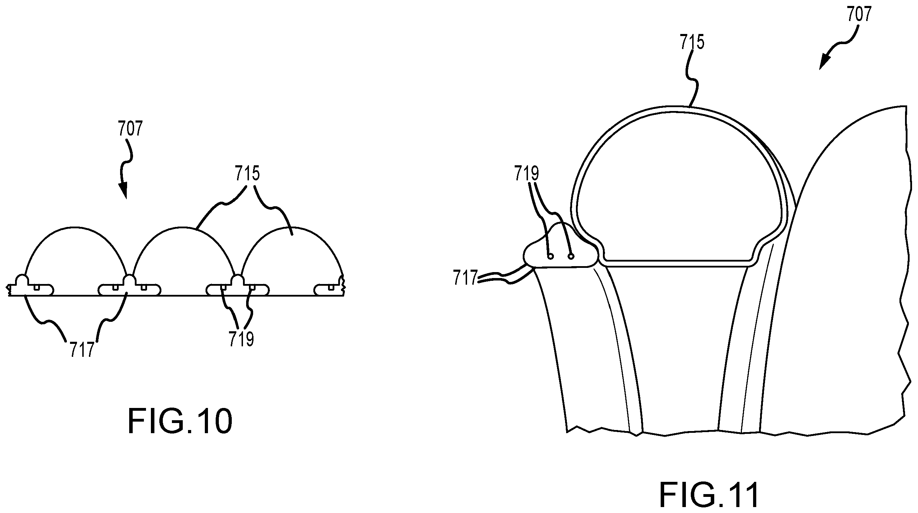

Referring to Figures 8 to 11, the inner tube 507, 607, 707 may additionally comprise one or more further spirally wound components in addition to the hollow bead 515, 615, 715. The additional spirally wound components may comprise one or more elongate structural components 517, 617, 717 which may optionally contain one or more heating elements and/or sensing elements, such as heating wires and/or sensing wires 719. In some forms, the elongate structural components 517, 617, 717 may comprise two heating elements and two sensing elements. In some forms, sensing wire(s) 719 connect to at least one sensor at the end of the tube. The sensor may be a thermistor or any other useful sensor to provide feedback about the environment within the tube. The heating wire(s) act as a heating element to prevent condensation forming in the tube from the humidified gas flow through the tube. For example, the inner tube may comprise a conduit such as the one described in WO 2012/164407, WO2014/088430 and shown in Figures 10 and 11.

A sheath 113 surrounds the outer surface of the inner tube 107, along at least a portion of the length L of the tube 107. The sheath 113 is tightly fitted over the inner tube 107 such that the sheath is biased towards or tensioned towards the longitudinal axis AA of the inner tube 107 and portions of an inner surface of the sheath contact the outer surface of the tube ridges to at least partially adopt the profile (which is preferably contoured) of the ridges. Portions of the sheath 113 that lie between the tube ridges 109 extend across the grooves 111 of the inner tube 107 and form bridging portion 125 between the ridges 109. The bridging portion 125 preferably comprises an inwardly curved (having an arc) profile. The bridging portions are spaced away from the grooves 211 of the tube 107 to define a cavity between the sheath 113 and tube 107 and between adjacent ridges 109. The sheath 113 is contoured as described in more detail below, such that it provides an outer surface for the conduit that is smoother than the outer surface of the inner tube, and that can be wiped down easily, thereby improving the ease at which the conduit 101 can be cleaned. The tight-fitting sheath 113 is flexible and is configured such that it does not significantly adversely impact the flexibility or performance of the conduit 101 in use.

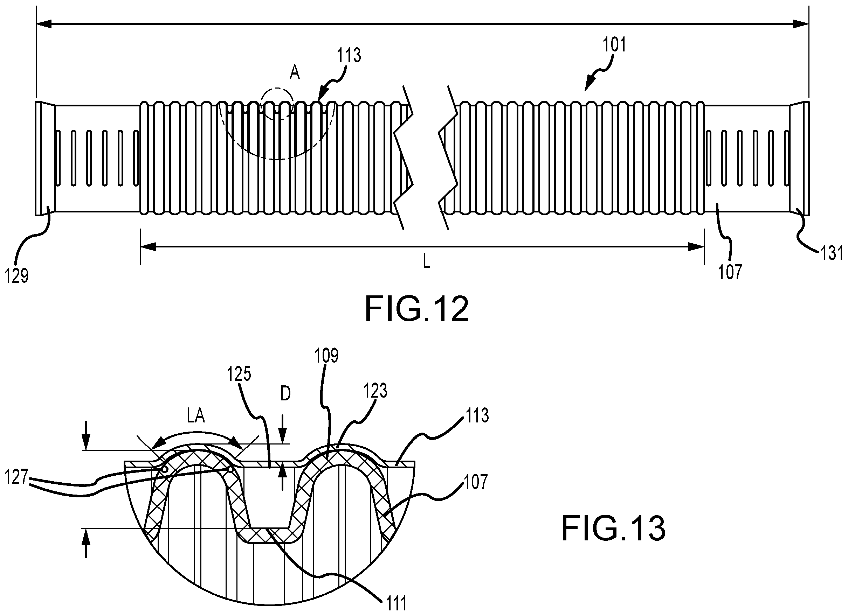

The sheath 113 comprises alternating attachment portions 123 that contact the ridges 109 of the inner tube 101, and bridging portions 125 that extend between adjacent attachment portions 123, over the grooves 111 of the inner tube 107. The attachment portions 123 contact the outer surface of at least a portion of the tube ridges 109, extending at least over the apex of the ridge 109a and optionally partly down the sides 109b of each ridge. The attachment portions 123 have a cross-sectional profile with a predetermined portion length LA between two transition regions 127. The transition regions 127 are regions where each attachment portion 123 of the sheath 113 transitions to the respective adjacent bridging portions 125. The transition regions 127 are located substantially symmetrically on the ridge relative to a plane through the centreline of the ridge apex 109a. As shown in Figure 13, the attachment portion length LA is measured along the surface of the attachment portion generally in the longitudinal direction but following the profile of the ridge 109.

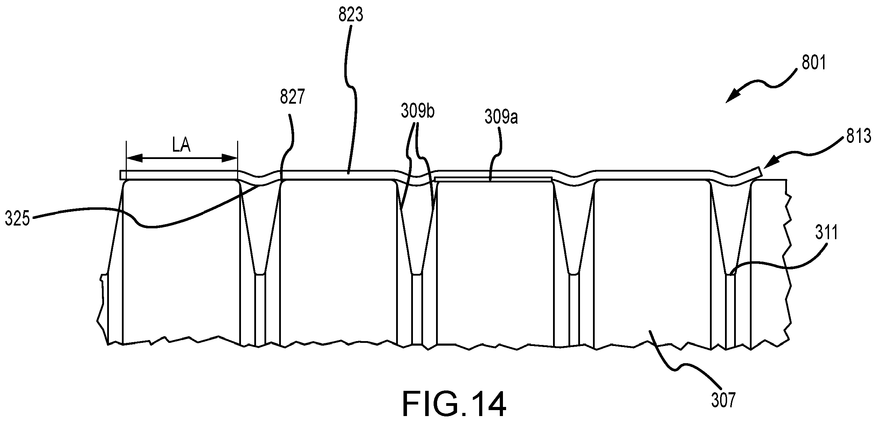

In the embodiments shown, the attachment portions 123 closely follow the profile of the respective inner tube ridge 109. For example, in the embodiment shown in Figure 13, the attachment portions 123 and the ridges of the tube 107 that are contacted by the attachment portions 123 are arcuate, with a convex outer surface, and the predetermined attachment length LA is an arc length. Other profile shapes are possible depending on the ridge shape of the inner tube 107. For example, in the embodiment of Figure 14, the attachment portion 123 has a substantially flat or blunt region to sit against the flat or blunt profile of the corresponding ridge 109, when viewed from the longitudinal cross-section of the conduit. The attachment portions 109 preferably have a profile with an outer surface that is convex, or that includes convex portions and is without concavities.

In some forms, at least a portion of the sheath 113 has a diameter that is smaller than the largest diameter of the tube 107. That is, portions of the sheath have a sheath diameter that is less than the maximum outer diameter of the inner tube 107 as defined by the apex of the ridges 109. For example, in some embodiments, such portions may have a sheath diameter of about 1mm less than the maximum outer diameter of the inner tube 107. But in other embodiments, the sheath diameter may be more than 1 mm less than the maximum outer diameter of the inner tube 107. The sheath diameter may be between about 10 mm and about 35 mm, preferably between about 10 mm and about 30 mm. In one embodiment, the sheath diameter is about 24 mm. In an alternative embodiment the sheath diameter is about 17 mm.

In some forms, this sheath diameter is formed when the sheath is cured after extrusion over the inner tube 107 or before being fit around the inner tube 107. When the sheath 113 is provided over the tube, it tightly fits around the tube, 'hugging' the ridges 109, such that attachment portions 123 of the sheath conform to the external surface of the ridges 109 of the tube. In portions of the sheath where the sheath passes over the ridges 109, the inner diameter of the sheath in those portions is substantially the same as the outer diameter of the ridges of the inner tube 107, and the outer diameter of the sheath accounts for the thickness of the sheath. In some forms, the ratio between the largest diameter of the tube, x, and the diameter of the outer sheath is between x:x and 0.9:x.

The sheath has a depth, D (see Figure 13), being the maximum distance in a radial direction between a point on the bridging portion and a point on one of the adjoining attachment portions That is, the radial distance between the maxima and minima of the sheath. The depth of the dip D may typically be greater than zero and less than about 15% of the outer diameter of the ridges of the inner tube 107, preferably less than about 10%. For example, in one embodiment tube having a maximum outer diameter of about 25 mm, the sheath has a dip of less than 2.5 mm.

In all forms, the attachment portions 123 contact the outer surface of the ridges 109 of the inner tube 107 and substantially remain in position, even as the conduit 101 flexes. Thus, the sheath 113 may be tightly fitted to the inner tube so that the attachment portions of the sheath substantially attach to the ridges under friction and/or tension, or the attachment portions may be attached to the ridges by other arrangements, such as through bonding, adherence, or using other suitable forms of attachment that substantially holds the sheath onto the tube in a tightly fitted arrangement. Typically, the attachment portions 123 attach to the ridge immediately adjacent a transition region 127 where the attachment portion 123 adjoins a respective adjacent bridging portion 125. However, optionally the attachment portions may additionally attach at other points along the length LA of the attachment portion 123, for example to the apex of the respective ridge. For some manufacturing methods, bonding substantially contiguously along the attachment portion length may be desired for ease of manufacturing.

The attachment portions 123 attach to the respective tube ridge 109 either by way of a snug fit whereby tensile forces in the sheath cause it to hug the ridge, and/or the attachment portions 123 may be bonded to the inner tube 107 at the respective tube ridge 109, for example, heat bonded or adhered to the inner tube.

Referring to the section view of Figure 14, the attachment portions 123 may be bonded to the respective inner tube ridge 109. In some forms the attachment portions 123 may be bonded to the respective inner tube ridge along substantially the length LA of the attachment portion 123 (measured in an axial direction). The bond may be contiguous along the length of the attachment portion LA. Alternatively, the bond may be intermittent in the axial direction, for example, it may only be in a discrete region at each edge of the attachment portion, immediately adjacent each transition region 127.

Further, the bond between the sheath attachment portions 213 and the inner tube ridge 107 preferably is contiguous in the annular or helical direction of the ridge.

The profile of the inner tube ridges 109 along with the location of the transition region 127 affects the ease and effectiveness of the bond of the sheath to the inner tube 107 at the transition region 127. Preferably, the transition regions 127 are positioned on or towards a side wall of the ridge 109b, such that they are closer to the longitudinal axis AA than the apex 109a of the respective ridge 109.

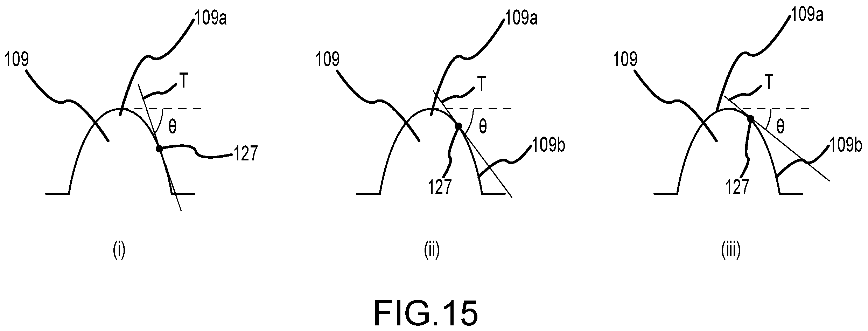

Referring to Figures 15(i) to 15(iii), at the transition region 127, the surface of the ridge 109 and sheath 113 define a tangent T. The tangent T has a predetermined angle that is selected to ensure a sufficient bond between the sheath 113 and inner tube 107, ease of manufacture, and/or to achieve the desired geometry of the bridging portions 125.

It is not desirable for the tangent T to have an angle θ that is too large or too small. Figures 15(i) to 15(iii) illustrate exemplary tangents T for three alternative transition region locations 127. If the angle 8 is too large (i.e. the tangent T is too steep), as illustrated in Figure 15(i), it may be difficult to obtain a sufficient bond or attachment between the sheath 113 and the inner tube 107 during manufacture. A smooth transition between the bridging portion 125 and the adjacent attachment portions 123 is desirable. A steeper tangent T may correspond to a larger angular inflection at the transition region 127 between the attachment portion 123 and the bridging portion 125. A larger angular inflection tends to mean that during flexing of the conduit 101, the force transmitted by the bridging portion 125 to the bond at the respective transition region 127 has a significant component in a direction normal to the ridge surface 109b at the transition region (as compared to a shear component). This normal component acts in a direction to urge the inner tube and sheath apart at the bond.

Conversely, if the tangent angle 8 is too small, as shown in Figure 15(iii), the bridging portions 125 may not have a sufficient depth D to ensure flexibility of the conduit 101, and/or the bridging portions 125 may have too much 'slack' making them prone to inverting so that they protrude outwards from the ribs 109 and away from the longitudinal axis AA. Such inverting is undesirable for cleaning as it can result in creasing or folds.

The desired angle 8 for the tangent T at the transition point 127 is expressed relative to the longitudinal axis AA of the conduit 101, such as between 10 and 85 degrees, 20 to 80 degrees or 30 to 75 degrees inclusive.

The angle of curvature of the inner tube ridge 109 between the apex portion 109a and the side walls 109b also impacts the ability of the sheath 113 to bond to the ridge 109 at the transition region 127. More angular ridges such as those shown in Figures 6 and 14 have a very small radius of curvature and, therefore, a sharp or sudden transition from the apex portion 309a to the side wall portion 309b. Depending on manufacturing techniques, it can be difficult to effectively bond the sheath to the sidewall portion 309b near this sharp transition, due to the melt strength of the coating material being stronger than the separation force that tends to occur with a sudden transition from the apex portion 309a to the side wall portion 309b.

The bridging portions 125 that extend from the attachment portions 123 at the respective transition regions 127, extend over the grooves 111 of the inner tube 107 to lie at a distance from outer surface of the grooves. The bridging portions 125 are spaced from the minima of the respective groove 111 such that at least a major portion of the bridging portion 125 is not in contact with the inner tube 107. The bridging portions 125 are spaced from and out of contact with the inner tube 107 along substantially all of the length of the bridging portions (in a longitudinal direction), with the bridging portion 125 only contacting the inner tube 109 at or near the transition regions 127, if at all, when the conduit is in a rest state.

Each bridging portion 125 has a span length SL that is the straight-line distance or cord length in the longitudinal direction between adjoining attachment portions 123 at the respective transition regions 127. Each bridging portion 125 has a maximum depth D taken as the maximum difference in the radius between the bridging portion 125 and the adjoining attachment portion 123. The maximum depth D of the bridging portions 125 is non-zero such that the outer diameter of the sheath 113 is undulating / varies along the length of the conduit 101.

In the embodiments shown, the maximum depth D is the distance in a radial direction from the minima on the bridging portion 125 to the maxima on an adjoining attachment portion 123, which coincides with the apex 109a of the respective ridge. That is, the outer diameter of the sheath 113 is smaller at the bridging portions than at the attachment portions 123. In alternative embodiments, the outer diameter of the sheath may be larger at the bridging portions 125 than at the attachment portions 123. That is, the bridging portions 125 may protrude outwards and have a convex outer surface.

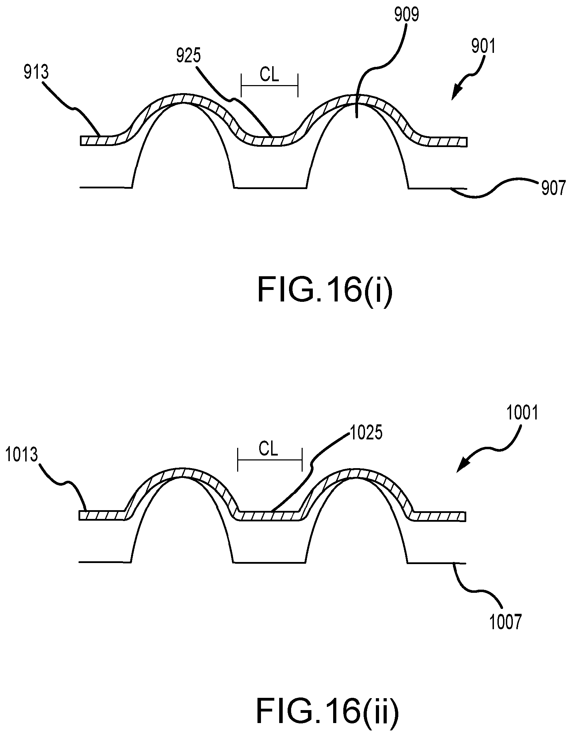

The depth D of the bridging portions 125 may be substantially unchanged along part of the length CL of the bridging portion such that an internal diameter of the bridging portion is substantially constant along that length, as shown in the embodiments of Figures 16(i) and 16(ii). In some embodiments, the depth D of the bridging portions 125 may be substantially unchanged along a major part of the span of the bridging portion, for example as illustrated in the embodiment of Figures 14 and 16(ii).

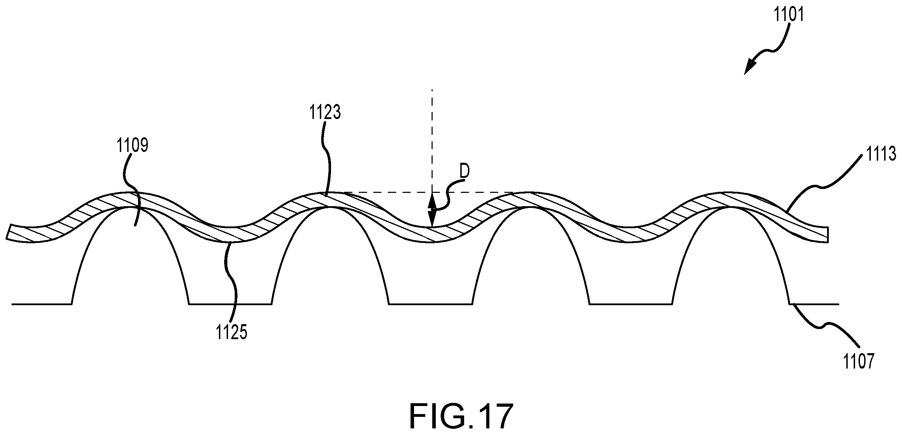

Alternatively, the bridging portion may be curved along its length with a maximum depth at a midpoint of the bridging portion, for example, as shown in the sinuous profile shown in the embodiment of Figure 17.

The depth D of the bridging portions 125 is less than the depth of the respective groove 111 over which the bridging portion extends in a spaced apart relationship. This spacing creates a hollow cavity between tube ridges 109, defined by the walls of the inner tube 107 and the groove 111, and the respective bridging portion of the sheath 113. Air is trapped in this cavity, forming an air pocket. These air pockets advantageously act to insulate the inner tube 107 from changes in ambient conditions and thereby to reduce heat loss from gases in the heated tube to the ambient environment.

In embodiments having annular ridges and grooves comprising a series of annular air pockets, each air pocket is formed between two adjacent ridges. This advantageously minimises any reduction of insulation or loss of performance of the conduit 101 if the sheath 113 is inadvertently punctured or otherwise compromised at an air pocket in one location, as the loss in insulation is localised. In contrast, in embodiments when the ridges are formed by one or more helical members, the air pocket(s) are elongate and spirally wound along the tube, with the number of air pockets corresponding to the number of ridge-forming helical members. Puncturing of the spirally wound air pocket at one location may result in a loss of insulation along the length of the conduit.

The depth and shape of the sheath bridging portions 125 is selected to enable ease of cleaning of the conduit 101. A smoother outer surface promotes easier cleaning and therefore is desirable. However, the sheath 113 should not significantly reduce the flexibility of the conduit 101. It is undesirable for the sheath to comprise sharp corners or radii and/or portions where the wall of the sheath is prone to folding or inverting to form localised creases or folds. Sharp corners and creases or folds can trap dirt or pathogens and make cleaning more difficult.

Factors such as the angle of curvature of the inner tube ridge 109 between the apex portion 109a and the side walls 109b, the span length SL of the bridging portion 125, the depth D and profile of the bridging portion 125, the location of the transition regions 127, and the flexibility of the sheath wall 113 all impact on the smoothness of the outer surface of the sheath and the tendency of the sheath bridging portions 125 to crease, fold, or buckle.

The span length SL of the bridging portion 125 influences the ability and tendency of the bridging portion 125 to invert / buckle outwards relative to the inner tube 107. A shorter span length reduces the potential for buckling/deflection of the bridging portion 125. Depending on the location of the transition regions 127 in the groove 111, this may make it more likely that the bridging portion 125 will remain in the groove 111, between ridges 109, when the conduit 101 is flexed and during cleaning. Conversely, a span length SL that is too large can increase the ability and tendency of the bridging portion 125 to invert / buckle outwards relative to the inner tube 107 to form folds or creases that may act as dirt traps.

The overall length of the bridging portion 125, measured along the contour of the bridging portion 125, compared to the span length SL may also determine the ability of the bridging portion to invert, buckle, or crease. Where the attachment portion 123 of the sheath applies tension to the respective bridging portions, this may minimise the likelihood of outwards buckling. In contrast, where the bridging portion 125 is much longer than the span length SL, there will be more 'slack' in the bridging portion, enabling more deflection or flex.

For a given inner tube, the bridging portion span length SL is influenced by the attachment length LA of the sheath attachment portions 123, and the pitch of the ridges of the inner tube 103. That is, for a given pitch P, a longer attachment length LA is associated with a shorter span length SL and conversely, a shorter attachment length LA is associated with a longer span length SL.

Similarly, for a given inner tube, a longer attachment length is associated with transition regions that are 'lower', that is, further from the ridge apex 109a and closer to the longitudinal axis LA of the tube. Lower transition regions reduce the likelihood of the bridging portions inverting / buckling outwards.

The depth D of the bridging portion 125 influences the manner in which the bridging portion is likely to deflect during flexing or cleaning of the conduit 101. The bridging portion preferably has a profile including a concave outer surface, with the maximum depth D being at or near a midpoint of the bridging portion 125, between adjacent ridges 109. This ensures that the bridging portion has a tendency to move inwards during any bending of the conduit 101 whilst cleaning, rather than outwards. In some forms, the gradient and/or curvature of the bridging portion 125 adjacent the transition regions 127 also influences how the bridging portion is likely to buckle or deflect during flexing or cleaning of the conduit 101, with a negative gradient ensuring the bridging portion 125 has a tendency to move inwards, rather than outwards. In other forms, the gradient and/or curvature adjacent the midpoint of the bridging portion 125 may influence the likelihood of and extent to which the bridging portion may buckle or deflect during flexing or cleaning of the conduit 101.

The combination of the span length SL of the bridging portion 125 and the depth D of the bridging portion 125 is a better predictor of the tendency of the sheath 113 to buckle or invert than either one of these parameters alone. The ratio of the bridging portion span length SL to its depth D is pre-determined to obtain the desired performance. The ratio is selected to reduce cavities, folds, or areas of the sheath 113 that would be difficult to reach, to minimise the risk of outwards buckling of the sheath, and thereby to promote ease of low level disinfection of the exterior surface of the sheath 113. However, the ratio should still be sufficient so as to not adversely impact the flexibility of the conduit 101. According to the invention, the predetermined ratio is between about 2:1 to about 2:0.1.

Figures 2 and 14 show two alternative forms of conduits 101, 801 with a sheath fitted to inner tube profiles. Figure 14 shows a sheath fitted to the inner tube of Figure 6. The transition from the ridge apex 309a to the side walls 309b of the ridges in the embodiment of Figures 6 and 14 is more angular, with a smaller radius of curvature than the curved ridges in the embodiment of Figure 2. Such an angular embodiment is less desirable because the sheath 813 has more of a tendency to buckle outwards during bending and cleaning compared to the deeper bridging portions 125 in the embodiment 101 of Figure 2. This tendency to buckle outwards is due to the transition regions 827 being located at a distance from the longitudinal axis AA nearer to the distance to the ridge apex 809a, and the short distance between the side surfaces 109b adjacent ridges.

In preferred embodiments of the conduit 101, the attachment length LA of the attachment portion 123 is more than 0 mm and less than or equal to about 4 mm, preferably between about 2 mm and about 4 mm. In the embodiment shown in Figure 12, the attachment length is about 2.5 - 3mm.

In preferred embodiments of the conduit 101, the span length of the sheath bridging portions 125 is more than 0 mm and less than or equal to about 3 mm, preferably between about 1 mm and about 2.5 mm. In the embodiment shown in Figure 12, the span length SL is about 2 mm.

In preferred embodiments of the conduit 101, the sheath bridging portions 125 have a maximum depth D of more than 0 mm and less than or equal to about 2 mm, preferably about 0.1 mm and about 1 mm. In the embodiment shown in Figure 12, the depth D is about 1 mm.

The conduit 101 is extendible and compressible in a longitudinal direction from a rest state and can flex laterally. As the distance between ridges increases, the depth of the grooves decreases and so too does the depth of the bridging portions. As the ridges and grooves flatten, the depth of the bridging portions decrease from the rest state and the bridging portions are tensioned. In some forms, the conduit is biased toward the rest stage so as to return to the rest state after being in the extended state.

The sheath is configured to withstand cleaning using specified cleaning chemicals.

The sheath 113 may comprise material that allows the sheath to stretch in the longitudinal direction, for example, the sheath may have a yield strain of at least 45% in the longitudinal direction. The wall thickness of the sheath is selected to facilitate extension of the sheath while minimising the likelihood of the sheath crinkling and any adverse impact on the performance of the tube. A wall thickness that is too thin can crinkle or folds can form in the bridging portions of the sheath, which can act as dirt traps. Thinner walls are also less durable to withstand repeated cleaning and use. A wall thickness that is too thick can result in a conduit that is too stiff and is therefore difficult to flex, and/or which applies tension to other components of the respiratory system such as the patient interface and may cause the tube to be difficult to arrange and setup. The thickness of the sheath is selected such that it is thin enough to facilitate extension and flexing of the sheath while minimising the likelihood of the sheath crinkling. Alternatively or additionally, the thickness of the sheath may be selected to be sufficiently durable enough to withstand multiple instances of cleaning, including cleaning with chemicals that are specified or expected to be used to clean the conduit. In preferred embodiments, the sheath has a substantially uniform wall thickness in a rest state of between about 20 µm and about 250 µm, preferably between about 120 µm and about 170 µm.

The sheath and/or the tube may comprise a low-density polyethylene (LDPE). However, other suitable, flexible thermoplastics may be used. The sheath and inner tube may comprise the same material or different materials. For some manufacturing methods, such as the one described below, it is advantageous for the sheath to comprise the same material as the inner tube for improved bonding of the sheath to the inner tube.

The material of at least the sheath should be one that is resistant to cleaning chemicals that are specified or expected to be used to clean the conduit, to ensure that the integrity of the conduit is not adversely affected by disinfection of the conduit.

Optionally, the material may also be heat resistant at the anticipated operating temperatures such that the properties of the conduit, including the integrity of any bonds between the inner tube and the sheath, do not change significantly when the tube is heated, for example, by heating wires/elements within the inner tube.

The sheath material and/or the inner tube material may be inherently anti-microbial and/or hydrophobic or may have an anti-microbial and/or hydrophobic surface treatment to deter attachment of contaminants to the sleeve surface and/or to aid infection control by killing or preventing bacteria growth.

The material of the sheath is preferably one with low tackiness to prevent the conduit 101 sticking to itself during use or sticking to a clinician's gloves or a cleaning cloth during cleaning or in use. The material of the sheath may inherently have this property or the outer surface of the sheath may be surface treated to reduce tackiness. In some forms, the coefficient of friction of the sheath material is between 0 to 0.6 and is preferably less than 0.3.

The outer surface of the sheath is preferably smooth. In some forms, a portion of the sheath that is intended to be cleaned is smooth.

The sheath and inner tube may be transparent or opaque. A transparent sheath and inner tube advantageously enables a clinician to observe any condensate formation in the tube.

To manufacture the conduit described above, the inner tube is formed having an outer surface with alternate ridges and grooves along at least a length of the tube, for example, formed by corrugations or one or more spirally wound wall components (or members as described elsewhere in the specification). For embodiments having a corrugated inner tube, the ridges and grooves may be formed via vacuum corrugation or pressure corrugation. Tubes with helical components may be formed using manufacturing techniques that will be known in the art.

A sheath 113 is then extruded over the inner tube, for example by passing the tube through a cross-head die, at least along the length of the tube comprising the alternate ridges and grooves. In some forms, the sheath is formed to be tight fitting so as to at least partially wrap around the ridges of the inner tube to form attachment portions over the ridges. In some forms, the sheath may optionally be heated to bond to the ridges of the tube. In some forms, the sheath may optionally be heated to shrink such that the sheath is tensioned around the tube. Alternatively, the sheath may be formed around the tube under a vacuum or pressure to seal/bond the sheath to the ridges of the tube. Alternatively, the sheath may be formed under a vacuum or pressure, and the sheath may be heated to seal/bond the sheath to the ridges of the tube.

A connector is attached or formed to one or each end of the conduit 101, for example by way of a press fit tapered connection, by clamping the connector to the conduit, or by way of a threaded connection, by adhering the connector to the conduit, or by overmoulding without damaging the integrity of the sheath 113.

In some embodiments, where a filter is used, the respiratory conduit 101 may be reused for several patients over the course of a day, with other components in the respiratory circuit replaced for each patient. It is envisaged that the respiratory conduit may be replaced on a daily basis. The respiratory conduit may be supplied as part of a kit for a respiratory system along with one or more other components for the respiratory system. For example, such a kit may include at least one of a dry line 17 (a conduit that generally delivers 'dry' gases (without humidity) from a flow source), a humidification chamber 6, a patient interface 5, a clip to releasably secure the respiratory conduit to clothing or bedding, and/or a filter 13 in addition to the conduit 101.

**The** respiratory conduit 101 may be provided as part of a package that includes other components for multi-patient use within a single day. For example, a first package may comprise the dry line 17, humidification chamber 6and the respiratory conduit 101. One or more second packages containing one or more components for single patient use may be provided in the kit. For example, the second package may comprise a patient interface and/or a filter.

**The** patient interface may be a nasal cannula or other suitable non-sealing or sealing patient interface. **The** patient interface may include a filter, or a filter may be provided separately for connection to the patient interface between the interface and the conduit 101.

Preferred embodiments of the disclosure have been described by way of example only and modifications may be made thereto without departing from the scope of the disclosure. For example, the conduit above has been described as being applied to an inspiratory tube. However, in alternative embodiments, the conduit may be an expiratory tube, a dry line, an endotracheal tube or other tube for a respiratory system.

The present invention is defined by the appended claims.

## Claims

1. A respiratory conduit (101) comprising:
a hollow tube (107) having an inlet (103), an outlet (105), and a longitudinal axis (AA), and defining a fluid flow path; wherein, along at least a length of the tube, an outer surface of the tube comprises alternate ridges (109) and grooves (111); and
a sheath (113) surrounding at least a portion of the length of the tube, the sheath comprising alternate attachment portions (123) contacting the ridges of the tube and bridging portions (125) that extend across the grooves of the tube, wherein each bridging portion has a span length (SL) being the distance in the longitudinal direction between adjacent attachment portions, and a depth (D) being the maximum distance in a radial direction between a point on the bridging portion and a point on one of the adjoining attachment portions;
wherein the depth of the bridging portion is greater than zero; and
**characterized in that** the ratio of the bridging portion span length to its depth has a predetermined ratio between about 2:0.1 and 2:1.

2. A respiratory conduit as claimed in any preceding claim, wherein the conduit comprises a rest state, such that the conduit is not extended, flexed or compressed and
the sheath has a yield strain of at least 45% in the longitudinal direction.

3. A respiratory conduit as claimed in any preceding claim, wherein the point on the bridging portion where the depth is maximum is located at a middle point between adjacent ridges.

4. A respiratory conduit as claimed in any one of claims 1 and 2, wherein the depth of the bridging portion is substantially the same along substantially all of the span of the bridging portion.

5. A respiratory conduit as claimed in any preceding claim, wherein the bridging portions are substantially arcuate.

6. A respiratory conduit as claimed in any preceding claim, wherein the sheath is tightly fitted to the tube such that the attachment portions contact an outer surface of at least a portion of the tube ridges and at least partially adopt a profile of the tube ridges.

7. A respiratory conduit as claimed in any preceding claim, wherein the attachment portions are bonded to the tube at the tube ridges.

8. A respiratory conduit as claimed in any preceding claim, wherein the attachment portion has a predetermined length between two transition regions (127) where the attachment portion transitions to respective adjoining bridging portions and the predetermined attachment length is more than 0 mm and less than or equal to about 4 mm.

9. A respiratory conduit as claimed in any preceding claim, wherein the attachment portion has a predetermined length between two transition regions where the attachment portion transitions to respective adjoining bridging portions and
a tangent (T) to the outer surface of the attachment portion at each transition region has an
angle (θ) of between about 10 degrees and about 70 degrees relative to the longitudinal
axis of the tube.

10. A respiratory conduit as claimed in any preceding claim, wherein each ridge or a portion thereof has a predetermined radius of curvature greater than 0 mm and less than or equal to about 3 mm.

11. A respiratory conduit as claimed in any preceding claim, wherein
the sheath has a substantially uniform wall thickness in a rest state of between about 100 µm and about 250 µm.

12. A respiratory conduit as claimed in any preceding claim, wherein the ridges and grooves comprise a series of annular ridges and grooves.

13. A respiratory conduit as claimed in any one of claims 1 to 11, wherein the ridges and grooves are helical.

14. A respiratory conduit as claimed in claim 13, wherein the tube is formed from two or more spirally wound components.

15. A respiratory conduit as claimed in claim 14, wherein the two or more spirally wound components may comprise at least one elongate hollow body, and at least one elongate structural component such as a heating element, sensing element, or both heating and sensing elements.

## Patentansprüche

1. Beatmungsleitung (101), umfassend:
einen hohlen Schlauch (107), der einen Einlass (103), einen Auslass (105) und eine Längsachse (AA) aufweist und einen Fluidströmungsweg definiert;
wobei entlang mindestens einer Länge des Schlauchs eine Außenfläche des Schlauchs abwechselnde Rippen (109) und Rillen (111) umfasst; und
eine Hülle (113), die mindestens einen Abschnitt der Länge des Schlauchs umgibt, wobei die Hülle abwechselnde Befestigungsabschnitte (123), die die Rippen des Schlauchs berühren, und Brückenabschnitte (125) umfasst, die sich über die Rillen des Schlauchs erstrecken, wobei jeder Brückenabschnitt eine Spannweite (SL), die der Abstand in der Längsrichtung zwischen benachbarten Befestigungsabschnitten ist, und eine Tiefe (D) aufweist, die der maximale Abstand in einer radialen Richtung zwischen einem Punkt auf dem Brückenabschnitt und einem Punkt auf einem der angrenzenden Befestigungsabschnitte ist;
wobei die Tiefe des Überbrückungsabschnitts größer als null ist; und
**dadurch gekennzeichnet, dass**
das Verhältnis der Spannweite des Brückenabschnitts zu seiner Tiefe ein vorbestimmtes Verhältnis zwischen etwa 2:0,1 und 2:1 aufweist.

2. Beatmungsleitung nach einem der vorhergehenden Ansprüche, wobei die Leitung einen Ruhezustand umfasst, sodass die Leitung weder gedehnt noch gebogen oder zusammengedrückt wird, und die Hülle eine Streckdehnung von mindestens 45 % in der Längsrichtung aufweist.

3. Beatmungsleitung nach einem der vorhergehenden Ansprüche, wobei sich der Punkt auf dem Brückenabschnitt, an dem die Tiefe maximal ist, am Mittelpunkt zwischen benachbarten Rippen befindet.

4. Beatmungsleitung nach einem der Ansprüche 1 und 2, wobei die Tiefe des Brückenabschnitts im Wesentlichen entlang der gesamten Spannweite des Brückenabschnitts im Wesentlichen gleich ist.

5. Beatmungsleitung nach einem der vorhergehenden Ansprüche, wobei die Brückenabschnitte im Wesentlichen bogenförmig sind.

6. Beatmungsleitung nach einem der vorhergehenden Ansprüche, wobei die Hülle eng am Schlauch anliegt, sodass die Befestigungsabschnitte eine Außenfläche mindestens eines Abschnitts der Schlauchrippen berühren und zumindest teilweise das Profil der Schlauchrippen annehmen.

7. Beatmungsleitung nach einem der vorhergehenden Ansprüche, wobei die Befestigungsabschnitte an den Schlauchrippen an den Schlauch gebunden sind.

8. Beatmungsleitung nach einem der vorhergehenden Ansprüche, wobei der Befestigungsabschnitt eine vorbestimmte Länge zwischen zwei Übergangsregionen (127) aufweist, in denen der Befestigungsabschnitt in jeweilige angrenzende Brückenabschnitte übergeht, und die vorbestimmte Befestigungslänge mehr als 0 mm beträgt und kleiner oder gleich etwa 4 mm ist.

9. Beatmungsleitung nach einem der vorhergehenden Ansprüche, wobei der Befestigungsabschnitt eine vorbestimmte Länge zwischen zwei Übergangsregionen aufweist, in denen der Befestigungsabschnitt in jeweilige angrenzende Brückenabschnitte übergeht, und eine Tangente (T) an die Außenfläche des Befestigungsabschnitts in jeder Übergangsregion einen Winkel (θ) von zwischen etwa 10 Grad und etwa 70 Grad relativ zur Längsachse des Schlauchs aufweist.

10. Beatmungsleitung nach einem der vorhergehenden Ansprüche, wobei jede Rippe oder ein Abschnitt davon einen vorbestimmten Krümmungsradius aufweist, der größer als 0 mm und kleiner oder gleich etwa 3 mm ist.

11. Beatmungsleitung nach einem der vorhergehenden Ansprüche, wobei die Hülle in einem Ruhezustand eine im Wesentlichen gleichmäßige Wanddicke von zwischen etwa 100 µm und etwa 250 µm aufweist.

12. Beatmungsleitung nach einem der vorhergehenden Ansprüche, wobei die Rippen und Rillen eine Reihe von ringförmigen Rippen und Rippen umfassen.

13. Beatmungsleitung nach einem der Ansprüche 1 bis 11, wobei die Rippen und Rillen spiralförmig sind.

14. Beatmungsleitung nach Anspruch 13, wobei der Schlauch aus zwei oder mehr spiralförmig gewickelten Komponenten gebildet ist.

15. Beatmungsleitung nach Anspruch 14, wobei die zwei oder mehr spiralförmig gewickelten Komponenten mindestens einen länglichen Hohlkörper und mindestens eine längliche Strukturkomponente wie etwa ein Heizelement, ein Sensorelement oder sowohl ein Heiz- als auch ein Sensorelement umfassen können.

## Revendications

1. Conduit respiratoire (101) comprenant :
un tube creux (107) présentant une entrée (103), une sortie (105) et un axe longitudinal (AA), et définissant un chemin d'écoulement de fluide ;
dans lequel, sur au moins une longueur du tube, une surface externe du tube comprend des crêtes (109) et des rainures (111) alternées ; et
une gaine (113) entourant au moins une partie de la longueur du tube, la gaine comprenant des parties de fixation alternées (123) en contact avec les crêtes du tube et des parties de pontage (125) qui s'étendent à travers les rainures du tube, où chaque partie de pontage présente une longueur de portée (SL) qui est la distance dans la direction longitudinale entre les parties de fixation adjacentes, et une profondeur (D) qui est la distance maximale dans une direction radiale entre un point sur la partie de pontage et un point sur l'une des parties de fixation contiguës ;
dans lequel la profondeur de la partie de pontage est supérieure à zéro ; et
**caractérisé en ce que**
le rapport de la longueur de portée de partie de pontage sur sa profondeur présente un rapport prédéterminé compris entre environ 2:0,1 et 2:1.

2. Conduit respiratoire selon une quelconque revendication précédente, dans lequel le conduit comprend un état de repos, de sorte que le conduit ne soit pas étendu, fléchi ou comprimé et la gaine présente une déformation à la limite d'élasticité d'au moins 45% dans la direction longitudinale.

3. Conduit respiratoire selon une quelconque revendication précédente, dans lequel le point sur la partie de pontage où la profondeur est maximale est situé à un point médian entre les crêtes adjacentes.

4. Conduit respiratoire selon l'une quelconque des revendications 1 et 2, dans lequel la profondeur de la partie de pontage est sensiblement la même sur sensiblement toute la portée de la partie de pontage.

5. Conduit respiratoire selon une quelconque revendication précédente, dans lequel les parties de pontage sont sensiblement arquées.

6. Conduit respiratoire selon une quelconque revendication précédente, dans lequel la gaine est ajustée étroitement au tube de sorte que les parties de fixation entrent en contact avec une surface externe d'au moins une partie des crêtes de tube et adoptent au moins partiellement un profil des crêtes de tube.

7. Conduit respiratoire selon une quelconque revendication précédente, dans lequel les parties de fixation sont collées au tube au niveau des crêtes de tube.

8. Conduit respiratoire selon une quelconque revendication précédente, dans lequel la partie de fixation présente une longueur prédéterminée entre deux régions de transition (127) où la partie de fixation effectue une transition vers des parties de pontage contiguës respectives et la longueur de fixation prédéterminée est supérieure à 0 mm et inférieure ou égale à environ 4 mm.

9. Conduit respiratoire selon une quelconque revendication précédente, dans lequel la partie de fixation présente une longueur prédéterminée entre deux régions de transition où la partie de fixation effectue une transition vers des parties de pontage contiguës respectives et une tangente (T) à la surface externe de la partie de fixation au niveau de chaque région de transition présente un angle (θ) compris entre environ 10 degrés et environ 70 degrés par rapport à l'axe longitudinal du tube.

10. Conduit respiratoire selon une quelconque revendication précédente, dans lequel chaque crête ou une partie de celle-ci présente un rayon de courbure prédéterminé supérieur à 0 mm et inférieur ou égal à environ 3 mm.

11. Conduit respiratoire selon une quelconque revendication précédente, dans lequel la gaine présente une épaisseur de paroi sensiblement uniforme dans un état de repos comprise entre environ 100 µm et environ 250 µm.

12. Conduit respiratoire selon une quelconque revendication précédente, dans lequel les crêtes et les rainures comprennent une série de crêtes et de rainures annulaires.

13. Conduit respiratoire selon l'une quelconque des revendications 1 à 11, dans lequel les crêtes et les rainures sont hélicoïdales.

14. Conduit respiratoire selon la revendication 13, dans lequel le tube est formé de deux composants enroulés en spirale ou plus.

15. Conduit respiratoire selon la revendication 14, dans lequel les deux composants enroulés en spirale ou plus peuvent comprendre au moins un corps creux allongé et au moins un composant structurel allongé tel qu'un élément de chauffage, un élément de détection ou à la fois des éléments de chauffage et de détection.
